# EUROPÄISCHE PATENTSCHRIFT

(11) **EP 4 282 387 B1**
(45) Veröffentlichungstag und Bekanntmachung des Hinweises auf die Patenterteilung: **11.12.2024**
(21) Anmeldenummer: 22000138.2
(22) Anmeldetag: 25.05.2022
(51) Int. Cl.: A61F 5/453

(54) **SYSTEM ZUM ANLEGEN EINES URINALKONDOMS**
URINARY CONDOM APPLICATION SYSTEM
SYSTÈME DE MISE EN PLACE D'UN PRÉSERVATIF URINAIRE

(43) Veröffentlichungstag der Anmeldung: 29.11.2023
(73) Patentinhaber: Urisan GmbH, 40213 Düsseldorf (DE)
(72) Erfinder: Ehrensperger, Samuel, 40545 Düsseldorf (DE)
(74) Vertreter: Rausch, Michael

(56) Entgegenhaltungen:
- EP-B1- 3 346 962
- DE-B3- 102007 020 517
- US-A- 2 567 926
- US-A- 5 471 998

## Beschreibung

Die vorliegende Erfindung betrifft ein System zum Anlegen eines Urinalkondoms zur bestimmungsgemäßen Verwendung.

Eine mit zunehmender Überalterung der Gesellschaften ebenfalls zunehmende Behinderung ist die sogenannte Inkontinenz, auch als Blasenschwäche bezeichnet. Unkontrolliert auslaufende Flüssigkeit wird entweder mittels saugender Hilfsmittel aufgenommen, beispielsweise Windeln und dergleichen, oder abgeleitet.

Zur Ableitung werden Katheter verwendet, die entweder durch die Bauchdecke direkt in die Blase eingesetzt werden, was häufig zu Infektionen führen kann, oder durch die Harnröhre des Penis in die Blase geschoben werden und zu Verletzungen an der Harnröhre führen können.

Eine Alternative sind sogenannten Urinalkondome, die eine Verbindung mit einem abführenden Schlauch, mit Auffangbeuteln und dergleichen ermöglichen.

Für die Versorgung der männlichen Harninkontinenz stellen Urinalkondome eine bevorzugte Alternative dar. Ein Nachteil besteht darin, dass das Anlegen eines Urinalkondoms schwierig sein kann. Bei eingeschränkter Fingerfertigkeit muss dies durch Fremdpersonen, Pflegepersonal oder dergleichen erfolgen. Aus unterschiedlichsten Gründen erweist sich dabei das ein Kondom aufnehmende Glied oftmals als wenig aufnahmefähig. Dies führt häufig zu Undichtigkeiten oder Faltungen, schlechten Sitz und dergleichen. Betroffene Patienten mit retrahiertem Penis oder mit Impotenz ist es bisher überhaupt nicht möglich, Urinalkondome anzusetzen und zu benutzen.

Aus dem Stand der Technik ist aus der CH 672 591 A5 eine Vorrichtung zum Ansetzen eines Urinals bekannt. Die Vorrichtung ermöglicht das vereinfachte Ansetzen eines Urinals, in dem die Penis-Manschette des Urinals über den Rand der Öffnung eines becherförmigen Gefäßes dieser Vorrichtung gestülpt wird und im Inneren dieses Gefäßes ein Unterdruck erzeugt wird.

Dadurch bläht sich die am Auslassende verschlossene Manschette auf und erlaubt einen problemlosen Zugang in das Innere.

Aus dem EP 3346962 B1, welches den nächsten Stand der Technik darstellt, ist eine gattungsgemäße Vorrichtung zum Anlegen eines Urinalkondoms zur bestimmungsgemäßen Verwendung bekannt, welche das Anlegen eines Urinalkondoms generell erheblich vereinfacht. Die bekannte Vorrichtung umfasst eine Aufnahmeglocke, eine Vakuumpumpe und eine Verbindungseinheit zur strömungstechnischen Verbindung der Aufnahmeglocke mit der Vakuumpumpe. Als Aufnahmeglocke wird ein topfförmiger Vorrichtungsteil bezeichnet, in welchen ein Urinalkondom einsetzbar ist. Urinalkondome liegen in der Regel in aufgerollter Form vor. Sie haben an der Spitze einen schlauchartigen Ansatz. Die Aufnahmeglocke wird so ausgestaltet, dass sie für die jeweilige Größe eines Urinalkondoms eine Möglichkeit zum Einlegen des Kondoms bietet, welches sich an die glockenförmige innere Oberfläche der Aufnahmevorrichtung anlegt. Der schlauchartige Ansatz ragt durch eine Öffnung an der entsprechenden Stelle der Aufnahmeglocke heraus. Die Verbindungseinheit hat einen rohrförmigen Kanal, so dass dann, wenn die Aufnahmeglocke mit der Verbindungseinheit verbunden ist, der Schlauchansatz eines eingelegten Kondoms in den Kanal ragt. Die Verbindungseinheit ist wiederum mit einer Vorrichtung zur Erzeugung eines Unterdruckes verbunden. Für die bestimmungsgemäße Verwendung wird in die Aufnahmeglocke ein Urinalkondom derart eingesetzt, dass der Schlauchansatz durch eine untere Öffnung der Aufnahmeglocke in den rohrförmigen Kanal der Verbindungseinheit ragt und der Kondomring an der freien Oberkante der Aufnahmeglocke zum Anliegen kommt. Der dazwischenliegende Kondombereich legt sich an den glockenförmig ausgebildeten Innenbereich der Aufnahmeglocke an. Die so vorbereitete Vorrichtung wird nun bestimmungsgemäß an einen Penis herangeführt und dieser wird aufgrund des durch den Betrieb der Vakuumpumpe erzeugten Unterdrucks in den an der Aufnahmeglocke anliegenden Bereich des Urinalkondoms eingesogen. Durch Abschalten der Vakuumpumpe kann nun die Vorrichtung abgenommen werden, so dass das Kondom am Glied verbleibt. Es kann nun in bestimmungsgemäßer Weise einfach abgerollt werden und ist somit optimal angesetzt. Auch ist ein Abnehmen der Vorrichtung bei laufender Vakuumpumpe, die unter Umständen regelbar ist, möglich, so dass in bestimmten Fällen auch ein faltenfreies Abrollen des Urinalkondoms unterstützt wird.

Bei der praktischen Anwendung hat sich herausgestellt, dass es im Bereich zwischen der Verbindungseinheit einerseits und der Vorrichtung zur Erzeugung des Unterdrucks andererseits zu unterschiedlichen Problemen kommt. Entweder ist die Verbindung nicht dicht genug, so dass der erzeugte Unterdruck nicht ausreicht, das Kondom vollständig in die Glocke zu saugen, oder die Verbindung ist so dicht, dass die Pumpe nicht mehr von der Verbindungseinheit gelöst werden kann, weil sich beide aneinander festgesaugt haben.

Ausgehend vom vorbeschriebenen Stand der Technik liegt der vorliegenden Erfindung die **Aufgabe** zugrunde, ein System zum Anlegen eines Urinalkondoms der gattungsgemäßen Art in Bezug auf eine störungsfreie und sichere Benutzung zu verbessern, damit diese eine strömungsdichte Verbindung zwischen Vorrichtung zur Erzeugung eines Unterdruckes einerseits und der Verbindungseinheit andererseits sicherstellt, ohne die Lösbarkeit der beiden Einheiten voneinander zu behindern..

Zur technischen **Lösung** wird eine Vorrichtung mit den Merkmalen des Patentanspruches 1 vorgeschlagen. Weitere Vorteile und Merkmale ergeben sich aus den Unteransprüchen.

Das erfindungsgemäße System umfasst zum einen eine Vakuumpumpe und zum anderen eine Verbindungseinheit. Beide werden derart aneinandergesetzt, dass sie mit zueinander deckungsgleichen Oberflächen aneinander liegen. Wenigstens eine der beiden Oberflächen weist eine unebene Struktur auf. Auf Seiten der Vakuumpumpe mündet in der für die Verbindung vorgesehenen Oberfläche eine Saugöffnung. Auf Seiten der Verbindungseinheit mündet ein Saugkanal, der mit der Saugglocke bzw. Aufnahmeglocke verbunden ist. Werden die Vakuumpumpe und die Verbindungseinheit zusammengesetzt, kann der von der Vakuumpumpe erzeugte Unterdruck durch die Verbindungseinheit bis zur Aufnahmeglocke wirken. Um zu verhindern, dass sich die beiden aufeinanderliegenden Oberflächen aneinander festsaugen, wird wenigstens eine der beiden Oberflächen mit einer uneben Struktur versehen.

Gemäß einem weiteren vorteilhaften Vorschlag der Erfindung werden die beiden aufeinanderliegenden Oberflächen randseitig abgedichtet. Dies kann auf unterschiedliche Weise erreicht werden. Beispielsweise kann ein umlaufende Dichtring zwischen beiden Oberflächen für eine Abdichtung sorgen. Gemäß einem vorteilhaften Vorschlag der Erfindung ist eine der Oberflächen mit einem ringförmigen Dichtrand umgeben, in welchen die jeweils andere Vorrichtung einsetzbar ist, um ihre zur Verbindung vorgesehene Oberfläche auf die andere Oberfläche aufzulegen. Zusätzlich kann der dichte Rand gegenüber dem Gehäuse durch einen weiteren Dichtring abgedichtet sein.

Die Vorrichtung ihrerseits umfasst eine Aufnahmeglocke, eine Vakuumpumpe und eine Verbindungseinheit zur strömungstechnischen Verbindung der Aufnahmeglocke mit der Vakuumpumpe. Als Aufnahmeglocke wird ein topfförmiger Vorrichtungsteil bezeichnet, in welchen ein Urinalkondom einsetzbar ist. Urinalkondome liegen in der Regel in aufgerollter Form vor. Sie haben an der Spitze einen schlauchartigen Ansatz. Die Aufnahmeglocke wird so ausgestaltet, dass sie für die jeweilige Größe eines Urinalkondoms eine Möglichkeit zum Einlegen des Kondoms bietet, welches sich an die glockenförmige innere Oberfläche der Aufnahmevorrichtung anlegt. Der schlauchartige Ansatz ragt durch eine Öffnung an der entsprechenden Stelle der Aufnahmeglocke heraus.

Eine Verbindungseinheit hat einen rohrförmigen Kanal, so dass dann, wenn die Aufnahmeglocke mit der Verbindungseinheit verbunden ist, der Schlauchansatz eines eingelegten Kondoms in den Kanal ragt. Die Verbindungseinheit ist wiederum mit einer Vorrichtung zur Erzeugung eines Unterdruckes verbunden. Dies kann ein Pumpball oder eine Pumpschere sein, die manuell betätigt werden, oder eine Elektropumpe für den Netzbetrieb oder Batteriebetrieb. Im einfachsten Fall ist die Verbindungseinheit lediglich ein Schlauch, der an der Aufnahmeglocke derart befestigt ist, dass er den Schlauchansatz eines eingesetzten Kondoms aufnehmen kann und die Vakuumpumpe strömungstechnisch mit dem Innenraum der Aufnahmegtocke verbindet. Um das Anlegen zu optimieren, ist die Innenkontur der Aufnahmeglocke so ausgearbeitet, dass sie ein Urinalkondom einer vorgegebenen Größe passgenau aufnehmen kann. Dadurch wird Faltenbildung vermieden und ein optimales Ansetzen unterstützt. An der bestimmungsgemäß äußeren Oberkante, das heißt die freie Oberkante die nicht mit einer Verbindungseinheit verbunden wird, weist die Aufnahmeglocke gemäß einem vorteilhaften Vorschlage der Erfindung eine umlaufende Nut auf. Diese dient dem Zweck, den Kondomring, der sich durch das Aufrollen des Urinalkondoms ergibt, aufzunehmen, wenn das Urinalkondom in die Aufnahmeglocke eingesetzt wird. Im Falle, dass keine Nut ausgebildet ist, ist erfindungsgemäß auch vorgesehen, dass der Kondomring an den Rändern der Aufnahmeglocke übergestülpt oder beispielsweise mit einem Klemmring befestigt wird.

In vorteilhafter Weise ist die Aufnahmeglocke außenseitig zylindrisch, um für eine gute Handhabung eine entsprechende Haptik bereitzustellen. Um für unterschiedliche Urinalkondome einsetzbar zu sein, kann die Aufnahmeglocke austauschbar mit der Verbindungseinheit verbunden werden. Auf diese Weise kann die Verbindungseinheit mit Aufnahmeglocken unterschiedlicher Größe und Ausbildung verbindbar sein.

Die gesamte Vorrichtung kann mit einem integrierenden Gehäuse versehen sein, in welches beispielsweise eine batteriebetriebene Elektropumpe, ein Verbindungskanal und eine Aufnahmeglocke untergebracht sind, wobei die Aufnahmeglocke in einem lösbaren Gehäuseteil angeordnet sein kann.

Für die bestimmungsgemäße Verwendung wird in die Aufnahmeglocke ein Urinalkondom derart eingesetzt, dass der Schlauchansatz durch eine untere Öffnung der Aufnahmeglocke in den rohrförmigen Kanal der Verbindungseinheit ragt und der Kondomring an der freien Oberkante der Aufnahmeglocke zum anliegen kommt. Der dazwischenliegende Kondombereich legt sich an den glockenförmig ausgebildeten Innenbereich der Aufnahmeglocke an. Die so vorbereitete Vorrichtung wird nun bestimmungsgemäß an einen Penis herangeführt und dieser wird aufgrund des durch den Betrieb der Vakuumpumpe erzeugten Unterdrucks in den an der Aufnahmeglocke anliegenden Bereich des Urinalkondoms eingesogen. Dabei werden Größen, Weichheiten und dergleichen durch einfaches Einsaugen kompensiert, so dass das Urinalkondom optimal angesetzt ist. Durch Abschalten der Vakuumpumpe kann nun die Vorrichtung abgenommen werden, so dass das Kondom am Glied verbleibt. Es kann nun in bestimmungsgemäßer Weise einfach abgerollt werden und ist somit optimal angesetzt. Auch ist ein Abnehmen der Vorrichtung bei laufender Vakuumpumpe, die unter Umständen regelbar ist, möglich, so dass in bestimmten Fällen auch ein faltenfreies Abrollen des Urinalkondoms unterstützt wird.

Mit der Erfindung wird eine einfach herstellbare und vor allem einfach anwendbare Vorrichtung bereitgestellt, mit welcher das Anlegen eines Urinalkondoms zur bestimmungsgemäßen Verwendung erheblich vereinfacht und optimiert wird.

Weitere Vorteile und Merkmale ergeben sich aus der folgenden Beschreibung anhand der Figuren. Dabei zeigen:
- Fig.1: eine schematische Darstellung eines typischen Urinalkondoms;
- Fig.2: eine schematische Darstellung einer Aufnahmeglocke und einer Verbindungseinheit;
- Fig.3: eine Darstellung gemäß Fig. 2 mit eingesetztem Urinalkondom;
- Fig.4: eine Darstellung des Ansetzvorganges der erfindungsgemäßen Vorrichtung zum Anlegen des Kondoms zur bestimmungsgemäßen Verwendung;
- Fig.5: eine Darstellung gemäß Fig. 4 in einer fortgeführten Position;
- Fig.6: eine Darstellung gemäß Fig. 4 in einer abschließenden Position;
- Fig.7: eine Darstellung des Urinalkondoms in bestimmungsgemäßer Position;
- Fig.8: eine Darstellung der Verbindungsoberflächen zwischen Vakuumpumpe und Verbindungseinheit, und
- Fig. 9: eine Darstellung des oberen Aufnahmebereiches einer Aufnahmeglocke.

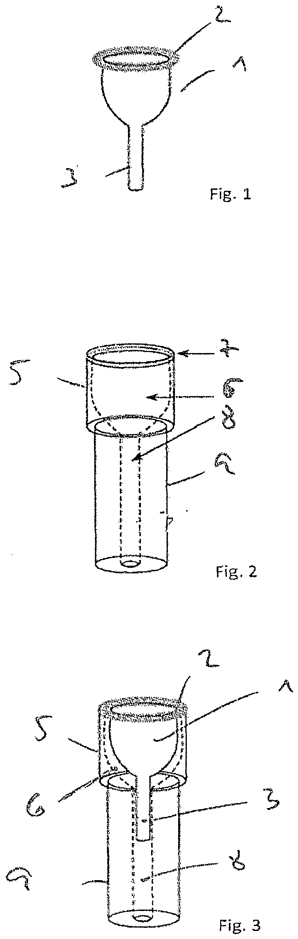

Fig. 1 zeigt ein typisches Urinalkondom 1. Dieses besteht aus einem elastischen Material und bildet eine Hülle, die ringförmig aufgerollt ist. Am vorderen Ende des Urinalkondoms befindet sich ein Schlauchansatz.

Die Aufwicklung 2 dient der Vereinfachung des Anlegens des Kondoms, der Schlauchansatz 3 der Abführung. In den Figuren 2 und 3 ist ein Ausführungsbeispiel für eine erfindungsgemäße Vorrichtung gezeigt. Eine Aufnahmeglocke 5 weist einen inneren glockenförmigen Hohlraum 6 auf. Dieser hat vorzugsweise eine Form, die der Aufnahme eines Urinalkondoms in der vorgesehenen Größe entspricht. Eine an der Oberkante umlaufende Nut 7 dient der Aufnahme des aufgewickelten Bereiches des Urinalkondoms, dem sogenannten Kondomring. Die Aufnahmeglocke 5 ist im Inneren strömungstechnisch verbunden mit einem rohrförmigen Kanal 8 der Verbindungseinheit 9. Die Aufnahmeglocke 5 und die Verbindungseinheit 9 sind außenseitig zylindrisch ausgebildet. Eine strömungsdichte Verbindung zwischen Aufnahmeglocke 5 und Verbindungseinheit 9 kann auf jede herkömmliche Weise hergestellt werden, verpressen, verschrauben, unter Verwendung von Dichtringen und dergleichen.

Wie insbesondere Fig. 3 zeigt, lässt sich in die Vorrichtung ein Urinalkondom derart einsetzen, dass der nicht aufgewickelte Bereich in der Aufnahmeglocke aufgenommen wird und der Schlauchansatz 3 in den rohrförmigen Kanal eingesetzt ist.

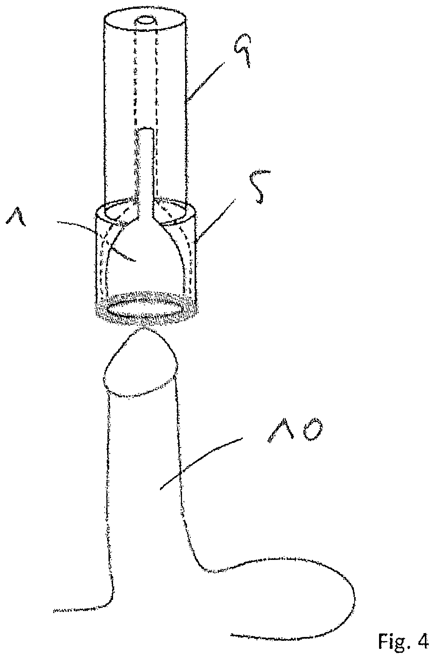

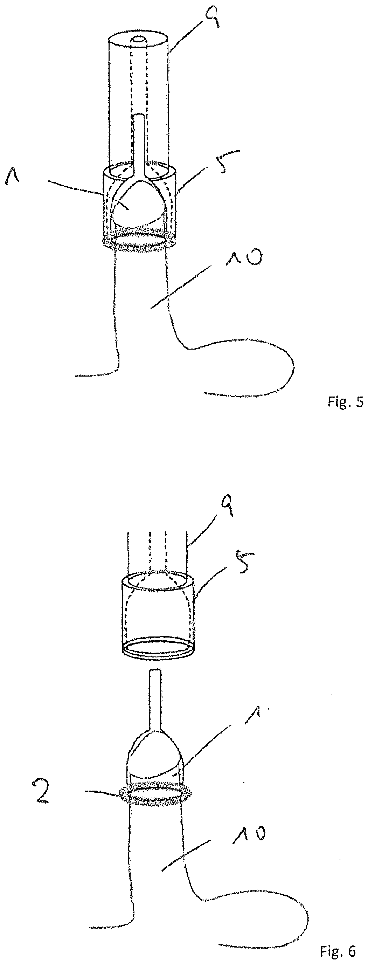

Die Applikation ist in den Figuren 4 bis 6 gezeigt. Die mit einer nicht gezeigten Vorrichtung zur Erzeugung eines Unterdrucks, Vakuumpumpe genannt, verbundene Verbindungseinheit stellt sicher, dass beim Aufsetzen der Vorrichtung auf einen Penis 10 das Urinalkondom 1 nach dem Aufsetzen auf die Spitze ein Einsaugen zulässt, während es sich selbst an der Aufnahmeglocke abstützt. Der Unterdruck kann manuell oder elektrisch erzeugt werden. Nach dem Einsaugen des Penis 10 in das Urinalkondom 1, wie dies in Fig. 5 gezeigt ist, kann die Vorrichtung abgenommen werden. Eine entsprechend höhere Haftreibung bewirkt, dass das Kondom aus der Aufnahmeglocke abgenommen wird. Dies kann durch Abschalten des Unterdrucks unterstützt werden.

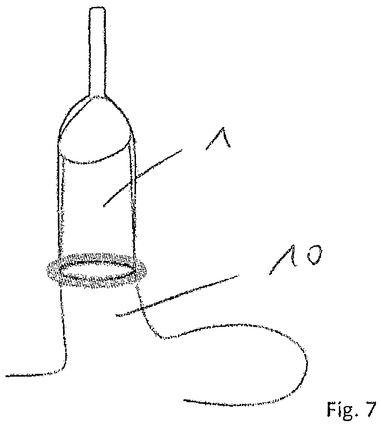

Wie in Fig. 7 gezeigt, ist nunmehr nur noch ein übliches Abrollen des aufgerollten Kondombereiches erforderlich.

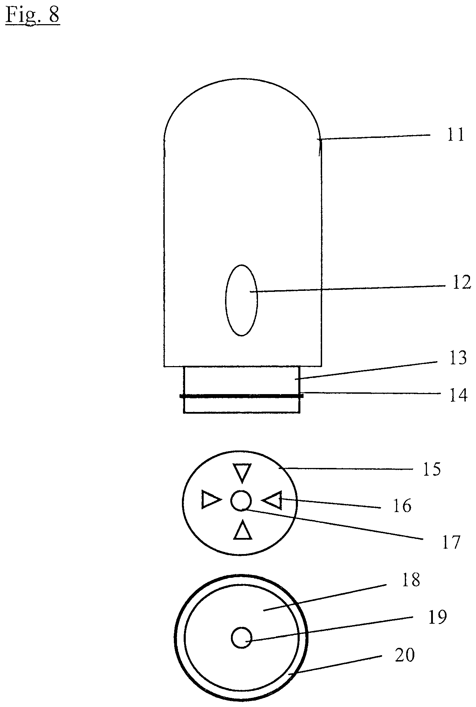

In Fig.8 ist das erfindungsgemäße Zusammenwirken der Elemente gezeigt. Im gezeigten Ausführungsbeispiel ist die Vakuumpumpe 11 als elektrische Pumpe dargestellt. Diese hat eine Bedienungseinheit 12. Damit lässt sich die Pumpe an und abschalten. An einem Ende weist die Pumpe einen Anschlussstutzen 13 auf, welcher im gezeigten Ausführungsbeispiel einen umlaufenden Dichtring 14 trägt. Die unter Ansicht der unteren Oberfläche 15 zeigt den Saugkanal 17 als mittlere Öffnung. Auf der Oberfläche 15 sind Erhebungen 16 ausgebildet. Diese können flach sein und ihre einzige Funktion besteht darin, ein direktes Ansaugen der aneinander liegenden Oberflächen zu verhindern.

An der Oberfläche 15 kommt die Oberfläche ab 18 der Verbindungseinheit zur Anlage. Diese weist die Öffnung 19 auf, die das Ende des Saugkanals der Verbindungseinheit darstellt. Im gezeigten Ausführungsbeispiel ist die Oberfläche 18 von einem Dichtrand 20 umgeben.

Im Zusammenwirken wird der Anschlussstutzen 13 der Pumpe in die durch den Dichterang 20 gebildete Röhre eingesteckt, bis die Oberfläche 15 auf der Oberfläche 18 zu liegen kommt. Sie sind lediglich durch die Abstandselemente 16 voneinander beanstandet. Die innere Wandlung des Dichtrandes 20 wirkt mit dem Dichtring 14 zusammen, so dass der von der Vakuumpumpe 11 erzeugte Unterdruck auf den Saugkanal der Verbindungseinheit über die Öffnung 19 übertragen wird.

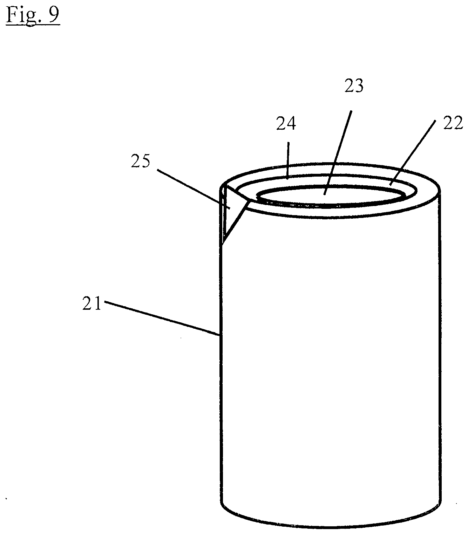

In Figur 9 ist der Aufnahmebereich einer Aufnahmeglocke 21 gezeigt. Den Luftkanal 23 umgebend ist an der inneren Oberkante eine umlaufende Phase oder Nut 22 ausgearbeitet, wodurch ein Aufnahmebereich 24 für den aufgewickelten Bereiches des Urinalkondoms, den sogenannten Kondomring, gebildet ist. Dieser Kondomring steht etwas über die Oberkante der Aufnahmeglocke 21 hinaus. Durch die Greifnut 25, eine Art Aussparung oder Senke im Bereich der äußeren Oberkante der Aufnahmeglocke 21 wird der Kondomring leichter erreichbar und ergreifbar. Im Bereich der Oberkante können eine Vielzahl von Greifnuten ausgebildet sein.

Das beschriebene Ausführungsbeispiel dient nur der Erläuterung und ist nicht beschränkend.

### Bezugszeichen

1 Urinalkondom
2 Aufwicklung
3 Schlauchansatz
5 Aufnahmeglocke
6 Hohlraum
7 Nut
8 rohrförmiger Kanal
9 Verbindungseinheit
10 Penis
11 Vakuumpumpe
12 Bedieneinheit
13 Anschluss-Stutzen
14 Dichtring
15 Anschluss-Stutzen untere Oberfläche
16 Abstandselemente
17 Saug-Kanal
18 Anschlussstelle Verbindungseinheit
19 Saug-Kanal
20 Dichtrand
21 Aufnahmeglocke
22 Nut
23 Kanal
24 Aufnahmebereich
25 Greifnut

## Patentansprüche

1. System zum Anlegen eines Urinalkondoms (1) zur bestimmungsgemäßen Verwendung, mit einer Aufnahmeglocke (5), einer Vakuumpumpe (11) und einer Verbindungseinheit (9) zur strömungstechnischen Verbindung der Aufnahmeglocke (5) mit der Vakuumpumpe, wobei die Verbindungseinheit (9) einen rohrförmigen Kanal (8) aufweist, mit dem die Aufnahmeglocke (5) strömungstechnisch verbunden ist, wobei der rohrförmige Kanal (8) aufnahmeglockenseitig als Aufnahme für einen Schlauchansatz des Urinalkondoms ausgebildet ist, dadurch gekennnzeichnet, dass die Verbindungseinheit an der mit der Vakuumpumpe zu verbindenden Seite eine Oberfläche (18) aufweist, welche zu einer an der Vakuumpumpe ausgebildeten Oberfläche (15) deckungsgleich ist, wobei wenigstens eine der beiden Oberflächen eine unebene Struktur aufweist, und die Vakuumpumpe und die Verbindungseinheit über die beiden aufeinanderliegenden Oberflächen strömungstechnisch verbunden sind.

2. System nach Anspruch 1, **dadurch gekennzeichnet, dass** die beiden aufeinanderliegenden Oberflächen randseitig abgedichtet sind.

3. System nach Anspruch 2, **dadurch gekennzeichnet, dass** eine der beiden Oberflächen der Verbindungseinheit oder der Vakuumpumpe durch einen Dichtrand (20) umgeben ist, in welchen das jeweils andere Element strömungsdicht einsetzbar ist.

4. System nach einem der vorhergehenden Ansprüche, **dadurch gekennzeichnet, dass** zwischen dem Dichtrand und der mit diesem zusammenwirkenden Oberfläche eine Dichtung (14) eingelegt ist.

5. System nach einem der vorhergehenden Ansprüche, **dadurch gekennzeichnet, dass** die Aufnahmeglocke (5) an einer Oberkante eine umlaufende Nut (7) aufweist.

6. System nach Anspruch 5, **dadurch gekennzeichnet, dass** die Aufnahmeglocke außenseitig im Bereich der Oberkante Greifnuten aufweist.

7. System nach einem der vorhergehenden Ansprüche, **dadurch gekennzeichnet, dass** die Aufnahmeglocke (5) außenseitig zylindrisch ist.

8. System nach einem der vorhergehenden Ansprüche, **dadurch gekennzeichnet, dass** die Aufnahmeglocke (5) lösbar mit der Verbindungseinheit (9) verbunden ist.

9. System nach einem der vorhergehenden Ansprüche, **dadurch gekennzeichnet, dass** die Verbindungseinheit (9) im Wesentlichen zylindrisch ausgearbeitet ist.

10. System nach einem der vorhergehenden Ansprüche, **dadurch gekennzeichnet, dass** die Aufnahmeglocke (5) und die Verbindungseinheit (9) aus Kunststoff bestehen.

## Claims

1. System for applying a urinal condom (1) for use as intended, having a receiving bell (5), a vacuum pump (11) and a connecting unit (9) for fluidically connecting the receiving bell (5) to the vacuum pump, wherein the connecting unit (9) has a tubular channel (8) to which the receiving bell (5) is fluidically connected, wherein the tubular channel (8) is formed on the receiving bell side as a receptacle for a hose attachment of the urinal condom, **characterized in that** the connecting unit has, on the side to be connected to the vacuum pump, a surface (18) which is congruent with a surface (15) formed on the vacuum pump, at least one of the two surfaces having an uneven structure, and the vacuum pump and the connecting unit being fluidically connected via the two superimposed surfaces.

2. System according to claim 1, **characterized in that** the two surfaces lying on top of each other are sealed at the edges.

3. System according to claim 2, **characterized in that** one of the two surfaces of the connecting unit or the vacuum pump is surrounded by a sealing edge (20) into which the respective other element can be inserted in a flow-tight manner.

4. System according to one of the preceding claims, **characterized in that** a seal (14) is inserted between the sealing edge and the surface interacting therewith.

5. System according to one of the preceding claims, **characterized in that** the receiving bell (5) has a circumferential groove (7) on an upper edge.

6. System according to claim 5, **characterized in that** the holding bell has gripping grooves on the outside in the area of the upper edge.

7. System according to one of the preceding claims, **characterized in that** the receiving bell (5) is cylindrical on the outside.

8. System according to one of the preceding claims, **characterized in that** the receiving bell (5) is detachably connected to the connecting unit (9).

9. System according to one of the preceding claims, **characterized in that** the connecting unit (9) is essentially cylindrical.

10. System according to one of the preceding claims, **characterized in that** the receiving bell (5) and the connecting unit (9) are made of plastic.

## Revendications

1. Système pour la mise en place d'un préservatif urinaire (1) pour une utilisation conforme, avec une cloche de réception (5), une pompe à vide (11) et une unité de liaison (9) pour la liaison fluidique de la cloche de réception (5) avec la pompe à vide, l'unité de liaison (9) présentant un canal tubulaire (8) avec lequel la cloche de réception (5) est reliée du point de vue de la technique d'écoulement, le canal tubulaire (8) étant conçu, du côté de la cloche de réception, comme un logement pour un embout de tuyau du préservatif urinaire, **caractérisé en ce que** l'unité de liaison présente, sur le côté à relier à la pompe à vide, une surface (18) qui coïncide avec une surface (15) formée sur la pompe à vide, au moins l'une des deux surfaces présentant une structure non plane, et la pompe à vide et l'unité de liaison étant reliées en termes d'écoulement par les deux surfaces superposées.

2. Système selon la revendication 1, **caractérisé en ce que** les deux surfaces superposées sont rendues étanches sur les bords.

3. Système selon la revendication 2, **caractérisé en ce que** l'une des deux surfaces de l'unité de liaison ou de la pompe à vide est entourée par un bord d'étanchéité (20) dans lequel l'autre élément respectif peut être inséré de manière étanche à l'écoulement.

4. Système selon l'une quelconque des revendications précédentes, **caractérisé en ce qu'**un joint d'étanchéité (14) est interposé entre le bord d'étanchéité et la surface qui coopère avec celui-ci.

5. Système selon l'une des revendications précédentes, **caractérisé en ce que** la cloche de réception (5) présente une rainure périphérique (7) sur un bord supérieur.

6. Système selon la revendication 5, **caractérisé en ce que** la cloche de réception présente des rainures de préhension du côté extérieur dans la zone du bord supérieur.

7. Système selon l'une des revendications précédentes, **caractérisé en ce que** la cloche de réception (5) est cylindrique à l'extérieur.

8. Système selon l'une des revendications précédentes, **caractérisé en ce que** la cloche de réception (5) est reliée de manière amovible à l'unité de liaison (9).

9. Système selon l'une quelconque des revendications précédentes, **caractérisé en ce que** l'unité de connexion (9) est réalisée de manière sensiblement cylindrique.

10. Système selon l'une des revendications précédentes, **caractérisé en ce que** la cloche de réception (5) et l'unité de liaison (9) sont en matière plastique.
